# EUROPEAN PATENT SPECIFICATION

(11) **EP 3 169 657 B1**
(45) Date of publication and mention of the grant of the patent: **12.09.2018**
(21) Application number: 15757336.1
(22) Date of filing: 14.07.2015
(51) Int. Cl.: C07C 29/62

(54) **PROCESS FOR THE PRODUCTION OF CHLOROHYDRINS FROM GLYCEROL AND ACYL CHLORIDES**
VERFAHREN ZUR HERSTELLUNG VON CHLORHYDRINEN AUS GLYCEROL UND ACYLCHLORIDEN
PROCÉDÉ POUR LA PRODUCTION DE CHLOROHYDRINES À PARTIR DE GLYCÉROL ET DE CHLORURES D'ACYLE

(30) Priority: 17.07.2014 IT RM20140395
(43) Date of publication of application: 24.05.2017
(73) Proprietor: Conser S.P.A., 00144 Rome (IT); Eurochem Engineering S.R.L., 20139 Milano (IT)
(72) Inventor: SANTACESARIA, Elio, I-20139 Milano (MI) (IT); DI SERIO, Martino, I-74013 Cava dei Tirreni (SA) (IT); TESSER, Riccardo, I-81100 Caserta (CE) (IT); VITIELLO, Rosa, I-84018 Scafati (SA) (IT)
(74) Representative: Sarpi, Maurizio
(86) International application number: PCT/IB2015/055313
(87) International publication number: WO 2016/009344

(56) References cited:
- US-A1- 2009 062 574
- E. SANTACESARIA ET AL: "New Process for Producing Epichlorohydrin via Glycerol Chlorination", INDUSTRIAL & ENGINEERING CHEMISTRY RESEARCH, vol. 49, no. 3, 3 February 2010 (2010-02-03), pages 964-970, XP055147341, ISSN: 0888-5885, DOI: 10.1021/ie900650x

## Description

### Technical field of the invention

The present invention relates to a new process for the production of chlorohydrins starting directly from glycerol and acyl chlorides. Acyl chlorides perform simultaneously the role of reagent and catalyst. Once they are introduced in a stoichiometric amounts, they react with glycerol to form esters and gaseous hydrochloric acid in amounts sufficient for producing α,γ-dichlorohydrin.

### Prior art

Glycerol is used in the field of fine chemistry, and its use is of particular importance for the production of α,γ-dichlorohydrin, which constitutes an intermediate product in the process of production of epichlorohydrin, which is in turn used for the synthesis of epoxy resins [1-7]. In the processes developed recently [8-11], to obtain α,γ-dichlorohydrin starting from glycerol, the latter is made to react with gaseous hydrochloric acid in the presence of a carboxylic acid, as catalyst, according to the following reaction:

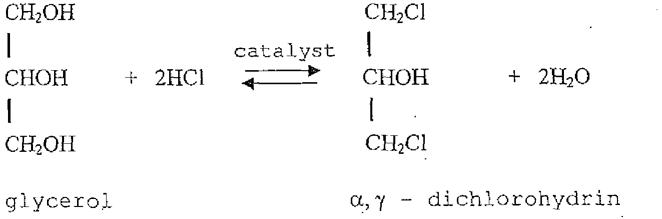

These processes, as may be noted, give rise to water as by-product, and this reduces, as has been observed, the rate of reaction.

In effect, the reaction proceeds through a first chlorination of glycerol, which leads to formation above all of α-monochlorohydrin and water, with small amounts of β-monochlorohydrih, which is followed by a second chlorination from which there are principally obtained the desired product, α,γ-dichlorohydrin, and very modest amounts of α,β-dichlorohydrin. The more detailed reaction scheme is consequently the following:

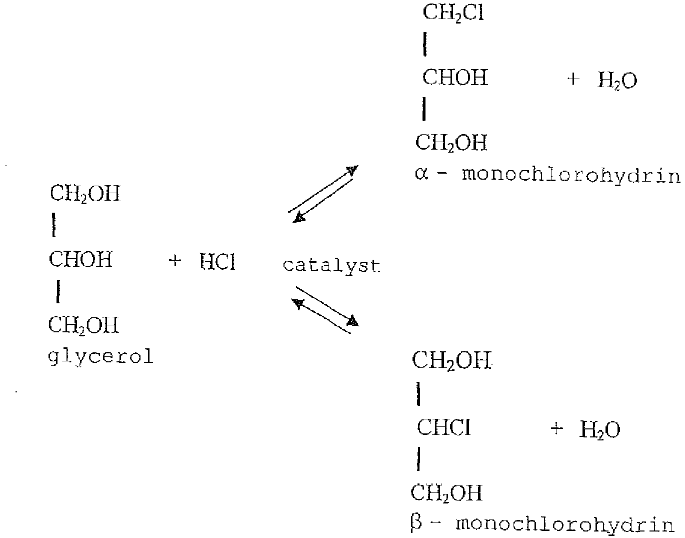

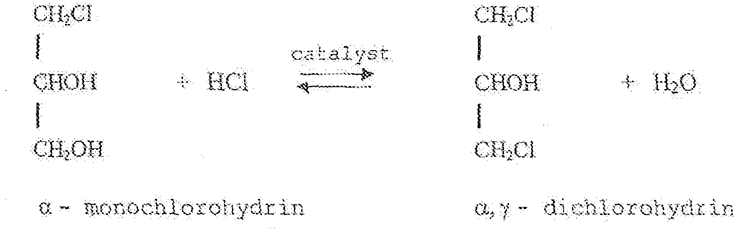

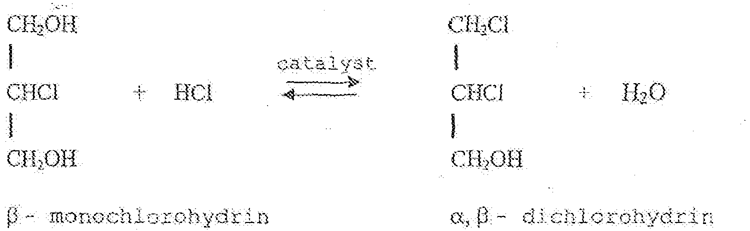

Traditional processes that used glycerol as raw material, which date back quite some time, envisaged reaction of glycerol with hydrochloric acid in aqueous solution, in the presence of acetic acid as catalyst, at a temperature of approximately 80-100°C [12-15].

Some patents describe processes which use an inert organic solvent that is immiscible, in which α,γ-dichlorohydrin is soluble, wherein the reaction is conducted at the boiling point of the reacting mixture, which hence varies according to the particular solvent used, but in any case does not exceed 110°C [16].

Other patents describe the reaction followed by complicated neutralizations, extractions, and distillations for recovery of α,γ-dichlorohydrin [17,18].

These processes ail present considerable drawbacks, such as:
- slowing-down of the reaction caused both by introduction of water into the reaction mixture, due to the use of aqueous hydrochloric acid, and by the failure to remove the water that is formed following upon the reaction; and
- difficult separation of α,γ-dichlorohydrin from the reaction mixture.

These drawbacks, together with the high cost of glycerol, as raw material, have prevented in the past this process from asserting itself. The process currently most widely adopted uses propylene as raw material, which yields 70% of α,β-dichlorohydrin and 30% of α,γ-dichlorohydrin in the mixture. Also this process presents drawbacks. In fact, the large amount of α,β-dichlorohydrin entails difficulties since this substance is much slower than α,γ-dichlorohydrin in yielding epichlorohydrin in the subsequent dehydrochlorination. This has repercussions on sizing of the plants and results in a reduction of the yields, with formation of by-products [5,6].

The reaction starting from glycerol would be potentially more advantageous provided obviously that glycerol is available at low cost. In this connection, it should be mentioned that the increasing production of biodiesel that gives rise to approximately 10 wt% of glycerol as by-product is causing in general an increasing availability and consequently a progressive reduction in the costs of glycerol. Moreover, since one of the major costs in the production of glycerol is its purification, it should be pointed out that the reaction of chlorination can take place directly on the raw, i.e., non-purified, glycerol with a further reduction in cost.

Recently, various patents have been published, which envisage the production of dichlorohydrins starting from glycerol and gaseous (anhydrous) hydrochloric acid. The patents all use carboxylic acids as catalysts and are distinguished from one another by the type of carboxylic acid used and/or by the operating conditions adopted.

One of the advantages of the production of chlorohydrins starting from glycerol, in the presence of carboxylic acids as catalysts, rather than from propene, is the high selectivity that is achieved in the production of α,γ-dichlorohydrin, which, as has already been said, is much more reactive than α,β-dichlorohydrin in forming epichlorohydrin. As a result, a considerable reduction in the volume of the dehydrochlorination reactor is possible or, given the same volume, an increase in productivity. In the patent proposed by Solvay [8], for example, use of adipic acid is suggested. The patent proposed by Eurochem Engineering [9] describes the use of the following carboxylic acids: propionic acid, malonic acid, levulinic acid, cytric acid, and succinic acid; moreover, the possibility of separating α,γ-dichlorohydrin by stripping is claimed. In the patent proposed by DOW Global Technologies [10] a considerable list of possible carboxylic-acid-based catalysts is suggested, but above all the advantage of operating under pressure is highlighted, given that the reaction rate and the yield are markedly affected by the partial pressure of HCl [19,20]. In the patent proposed by CONSER S.p.A. [11] there is suggested the possibility of using two different catalysts in sequence (first malic acid, and then succinic acid), starting the reaction at low pressure (1-2 bar) and increasing it in the second stage (8-10 bar).

The performance of carboxylic acids has been intensely studied [19, 21, 22], and a certain correlation has been found between the pKₐ of the acid, which should be comprised in the range 4-5, and the catalytic activity. The series, for example, acetic acid, mono-chloroacetic acid, and tri-chloroacetic acid manifests a decreasing activity since the acidic force increases with the chlorine content [22]. Some catalysts once again containing carboxylic-acid functional group have proven selective in producing monochlorohydrins since the subsequent chlorination is possible only after glycerol has been completely converted into monochlorohydrin [21-23]. A reaction mechanism has been formulated that is able to describe the kinetic behaviour observed experimentally [23].

Also these patents, however, present the drawback that as by-product water is formed in quite a considerable amount, and this causes a progressive slowing-down of the reaction. Recently, a patent has been published in which the use of a new class of catalysts is claimed that has never been experimented previously, constituted by acyl chlorides [24]. In this patent, emphasis is laid on how the use of acyl chlorides, given the same molar concentration, gives rise to activity and selectivity higher than those of traditional carboxylic-acid-based catalysts. From a study of the performance of these catalysts it has surprisingly been noted that they can give rise to the desired product of reaction, α,γ-dichlorohydrin, without having necessarily to supply hydrochloric acid, which is formed *in situ* following upon the reaction between glycerol and acyl chloride that brings about formation of ester. The hydrochloric acid then reacts with the ester to yield the product of reaction and carboxylic acid as by-product instead of water, as in the traditional process. Given that carboxylic acid is a good catalyst for the reaction, this proceeds quickly and with good selectivity, whereas water is known to have a negative action on the reaction speed.

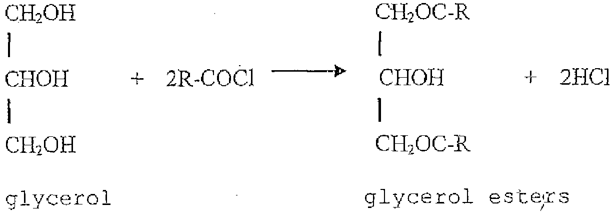

Hydrochloric acid then reacts with the ester to yield the reaction 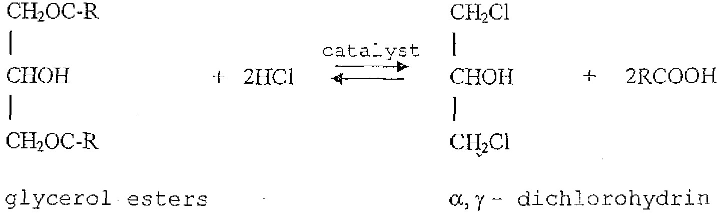 i.e., as a whole

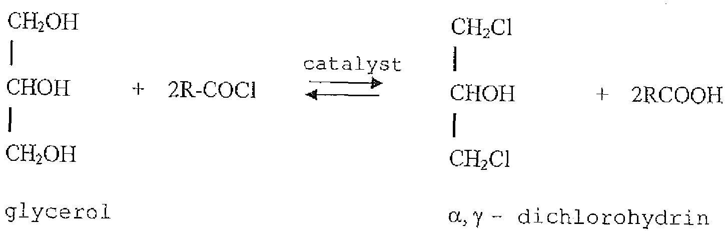

Using then glycerol/acyl chloride ratios of 1:2, prevalently α,γ-dichlorohydrin is obtained with the high reaction speed that characterizes the catalytic action already observed in the use of acyl chlorides as catalysts [24]. Carboxylic acid, which is a good catalyst for the reaction, in a high concentration is then formed instead of water, which has a negative effect on formation of esters and hence on the reaction rate.

### Summary of the invention

Consequently, forming a subject of the present invention is a process for the production of chlorohydrins from glycerol and acyl chlorides. The latter, used in a stoichiometric amount, act both as reagents and as catalysts. In fact, setting in contact an acyl chloride of the general formula RCOCl in a stoichiometric ratio of 1:2 with glycerol, the following reaction instantaneously takes place: 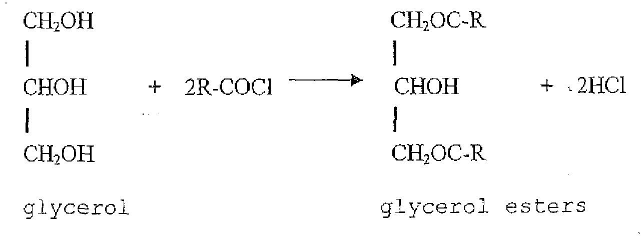 i.e., the hydrochloric acid necessary for the reaction of formation of chlorohydrins is formed in *situ* simultaneously with the glycerol esters. The hydrochloric acid formed then reacts with the esters to yield the reaction 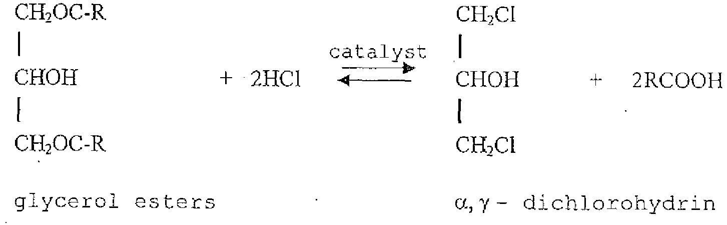 i.e., as a whole

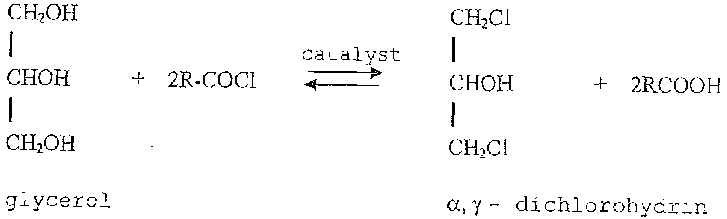

Using then glycerol/acyl chloride ratios of 1:2 α,γ-dichlorohydrin is prevalently obtained with the high reaction rate that characterizes the catalytic action already observed in the use of acyl chlorides as catalysts [24]. In this case, however, carboxylic acid is formed in a high concentration instead of water, as in traditional processes that are catalysed by carboxylic acids. Carboxylic acid has a good catalytic effect on the reaction, whereas water is known to have a negative effect on the formation of esters and hence on the reaction rate. The reaction hence takes place much faster and with good selectivity. Acyl chlorides that can be used have the general formula 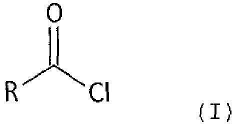 where R is a linear, branched, or cyclic alkyl chain with 2 to 10 carbon atoms, possibly substituted with one or more R' groups, which are the same as or different from one another, chosen from among: a linear, branched, or cyclic aliphatic group C₁-C₁₀; an aromatic group C₁-C₁₀; and one, or more than one, further acyl-chloride group.

Carboxylic acid, which is formed as by-product, can be separated, purified, and returned to the supplier of acyl chloride to be subjected to chlorination and then re-used, with evident economic advantages.

An advantage of this process is, in fact, represented by the fact that the desired reaction takes place without the need to have available gaseous hydrochloric acid at the site of production of the chlorohydrins. Another advantage is represented by a greater ease of continuous operation.

A further purpose of the present invention is the possibility of using also raw glycerol as by-product of biodiesel production, limiting the operations of preliminary treatment to: neutralization of the base used as catalyst with mineral acids, preferably hydrochloric acid, and elimination of the residual methanol and of any possible humidity.

Another purpose of the present invention is to provide a process that will enable a practically total conversion of glycerol.

Yet a further purpose of the present invention is to provide a process that will develop in thermodynamically favourable conditions, with a yield and a reaction rate that are accordingly increased.

The above and other purposes that will emerge more clearly from the ensuing description of the invention are achieved by a process for the production of chlorohydrins and in particular of α,γ-dichlorohydrin comprising the step of stoichiometric reaction between glycerol and an acyl chloride.

Other purposes of the invention will emerge clearly from the ensuing description of embodiments of the invention.

### Brief description of the drawings

To enable a better understanding, the description of the invention will be made with reference to the attached plates of drawings, which illustrate purely by way of non-limiting example a preferred embodiment thereof.

In the plates of drawings:
Figure 1 is a scheme of the plant used in the tests provided by way of example, where: designated by 1 is a Hastelloy reactor with a capacity of 300 mL; designated by 2 is an HCl cylinder; designated by 3 is a valve for drawing off the samples to be analysed; designated by 4 is a magnetic stirrer; designated by 5 is a temperature reader; designated by 6 is a pressure reader; and designated by 7 is a system for neutralizing the non-reacted HCl (two Drechsel bubblers in series containing a solution of sodium hydroxide);
Figure 2 is a graph that shows the evolution of the pressure in the reactor during and after supply of acetyl chloride according to the modalities of Example 1; and
Figure 3 is a graph that shows the evolution of the pressure in the reactor during and after supply of acetyl chloride according to the modalities of Example 2.

### Detailed description of the invention

The process according to the invention comprises the fundamental operation of causing glycerol to react with an acyl chloride, for example, acetyl chloride, in a stoichiometric ratio of 1:2. The reaction between glycerol and acetyl chloride is very fast already at room temperature but at 100-120°C is practically instantaneous. This means that, if the contact between the two reagents is complete right from the start, the pressure in the reactor increases considerably on account of the sudden development of HCl. It is hence expedient to add gradually one reagent to the other (preferably adding the acyl chloride to the glycerol). In this way, a moderate pressure value of 4-6 bar is maintained, and it is possible to adjust as desired the duration of the process regulating the flowrate of acyl chloride. The process according to the invention can hence be conducted in a semicontinuous reactor, with continuous supply of acyl chloride to the reactor as it is consumed.

The process can, however, be conducted also continuously, with continuous supply both of glycerol and of acyl chloride. The reaction temperature can be maintained in a range of 60-250°C, preferably between 100 and 175°C.

Acyl chlorides may be used that have the general formula indicated above. Acyl chlorides already used in a prior patent as catalysts are preferable, which may be monofunctional and contain 2 to 10 carbon atoms or else bifunctional or polyfunctional with 2 to 10 carbon atoms. Non-limiting examples of acyl chlorides that can be used in the present invention are: acetyl chloride, propionyl chloride, hexandioyl dichloride, propandioyl dichloride, butandioyl dichloride, phenyl acetyl chloride.

At a laboratory level and in the examples provided in the present description, acyl chloride was supplied with a pump in a reactor kept under stirring in which glycerol had been previously introduced. From this reactor, samples were taken at different times to follow the evolution in time of the reaction by determining, through chemical analysis, distribution of the products for the various samples taken.

In the examples provided hereinafter, purely by way of non-limiting illustration of the present invention, the tests for the production of chlorohydrins, in particular of α,γ-dichlorohydrin, conducted with different modalities are described in detail.

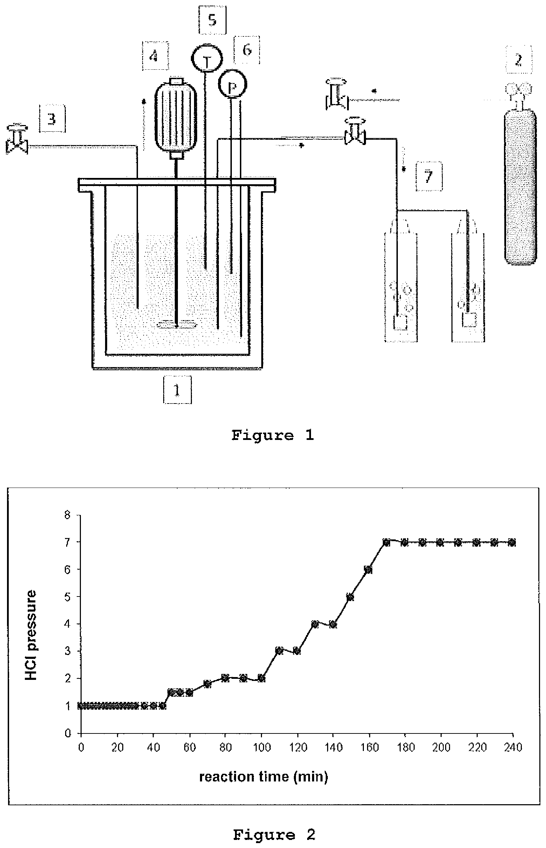

Figure 1 shows the scheme of the apparatuses used for carrying out the experiments provided in the examples. The reactor 1 is a 300-cm³ Parr autoclave heated from outside with a thermally regulated oven to achieve the pre-set temperature. Glycerol is introduced into the reactor in a predefined amount (for example, 100 g). A magnetic stirrer 4, normally kept at approximately 1000 r.p.m., enables the reaction mixture to be kept homogeneous. Then the reactor is heated until the reaction temperature is reached. Acyl chloride is then introduced at a pre-set constant flowrate (1-2 cm³/min), and supply is continued until the stoichiometric value is reached (acyl-chloride moles : glycerol moles = 2). At this point, supply of acyl chloride is interrupted, and the mixture is left to react for another few hours until the pressure of HCl in the reactor is found not to decrease any further. Small amounts of the reaction mixture are drawn off through the valve 3, at different reaction times, and analysed to determine the composition thereof. The temperature and pressure are read with the readers 5 and 6. The non-reacted HCl is discharged from the autoclave by causing it to pass in the Drechsel bubblers, which are filled with a sodium hydroxide saturated solution and contain an indicator that signals when neutrality has been reached. The results obtained are described in the following examples.

Described in Example 1 is the behaviour of the system obtained by supplying into the reactor of Figure 1, which contained 100 g of glycerol, acetyl chloride at a flowrate of 1 cm³/min for 154 min. The amount supplied of acetyl chloride corresponded to an acetyl-chloride/glycerol stoichiometric ratio of 2. The reaction temperature was kept at 100°C. The test was protracted for a total of 4 hours.

Described in Example 2 is the behaviour of the system obtained by supplying into the reactor of Figure 1, which contained 100 g of glycerol, acetyl chloride at a flowrate of 2 cm³/min for 80 min. The amount supplied of acetyl chloride corresponded to an acetyl-chloride/glycerol stoichiometric ratio of 2. The reaction temperature was kept at 100°C. The test was protracted for a total of 4 hours.

In Example 3 the reaction mixture obtained at the end of the two previous examples was subjected to distillation, separating in order: the residual hydrochloric acid, the acetic acid formed, and possible traces of water. The residue obtained from distillation was subjected to chemical analysis, which showed a preponderant formation of the desired product α,γ-dichlorohydrin.

### EXAMPLES

### Example 1- Reaction between glycerol and acetyl chloride by supplying the latter at a constant flowrate of 1 cm³/min

The test was conducted in the reactor of Figure 1 maintaining the following conditions: initial glycerol charge: 100 g; flowrate of acetyl chloride: 1 cm³/min (supplied at room temperature); duration of supply: 154 min; duration of test: 4 h; operating temperature: 100°C.

In these conditions, the pressure that is set up in the reactor owing to development of hydrochloric acid is the result of the kinetic balance between the reaction of formation of the esters and consequent formation of HCl and the reaction of formation of chlorohydrins with consumption of HCl. When supply of acetyl chloride ceased, the reaction was made to proceed for another 86 min. A residual pressure remained in the reactor, corresponding to a small amount of unreacted HCl. This HCl was released and abated by means of traps, and the liquid obtained after analysis on a small sample was kept for undergoing purification of the product (see Example 3).

Shown in Figure 2 is the plot of the pressure, within the reactor, during supply of acetyl chloride and for the subsequent duration of the test.

As may be noted from the graph of Figure 2, the reaction of formation of chlorohydrins was extremely fast considering that for approximately 80-100 minutes the pressure increase was practically negligible, i.e., all the hydrochloric acid that had formed reacted. Another observation is that, at the end of supply of the acetyl chloride, the HCl pressure settled at a value just above 7 bar without decreasing, this being a sign of the end of the reaction.

Table 1 shows the analyses of the samples taken, respectively, at 180 and 240 min:

**Table 1 - Analysis of the samples taken at different reaction times**

| Time | alpha | Beta | alpha gamma | alpha beta | glycerol |
|---|---|---|---|---|---|
| 0 | 0 | 0 | 0 | 0 | 100 |
| 180 | 1.8 | 8.2 | 81.4 | 8.6 | 0 |
| 240 | 0.5 | 5.9 | 82.3 | 11.3 | 0 |

These analyses confirm that, shortly after the stoichiometric amount of acetyl chloride is reached, the reaction can be considered as completed.

### Example 2 - Reaction between glycerol and acetyl chloride by supplying the latter at a constant flowrate of 2 cm³/min

The test was once again carried out in the reactor of Figure 1 maintaining the following conditions: initial glycerol charge:100 g; flowrate of acetyl chloride: 2 cm³/min (supplied at room temperature); duration of supply: 80 minutes; total duration of test: 4 h; operating temperature: 100°C.

The pressure that is set up in the reactor owing to development of hydrochloric acid is once again the result of the kinetic balance between the reaction of formation of the esters and consequent formation of HCl and the reaction of formation of chlorohydrins with consumption of HCl. When supply of acetyl chloride ceased, the reaction was made to proceed for another 160 min. Also in this case, a residual pressure remained in the reactor, corresponding to a small amount of non-reacted HCl. This HCl was released and abated by means of traps, and the liquid obtained after analysis on a small sample was kept for undergoing purification of the product (see Example 3).

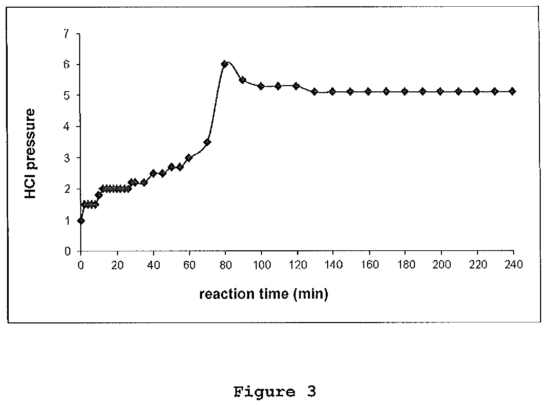

Figure 3 shows the plot of the pressure, within the reactor, during supply of acetyl chloride and throughout the duration of the test.

In this case, the addition of acetyl chloride was rather fast, and hence the pressure in the system rose more rapidly even though the final plateau remained lower, which is a sign that more hydrochloric acid was consumed. Another observation is that, at the end of supply of acetyl chloride, the HCl pressure settled at a value just above 5 bar without decreasing any further, this being a sign of the end of the reaction.

Appearing in Table 2 are the analyses of the samples taken, respectively, at 180 and 240 min.

**Table 2 - Analysis of the samples taken at different reaction times**

| Time | alpha | Beta | alpha gamma | alpha beta | glycerol |
|---|---|---|---|---|---|
| 0 | 0 | 0 | 0 | 0 | 100 |
| 180 | 0 | 4.0 | 91.9 | 4.1 | 0 |
| 240 | 0 | 3.7 | 92.3 | 4.0 | 0 |

Figure 3 suggests that, after 130 minutes, i.e., after the stoichiometric amount of acetyl chloride is reached, the HCl pressure in the reactor settles, and hence the reaction can be considered terminated. This test moreover indicates that it is possible to reduce the time of reaction by regulating appropriately operating conditions such as initial amount of glycerol, rate of addition of acyl chloride, and reaction temperature, whereas the pressure in the reactor will be self-regulated according to the preselected conditions. Moreover, operating in the way described, i.e., adding the reactive acetyl chloride faster, the selectivity to α,γ-dichlorohydrin improves.

### Example 3 - Purification of the product of reaction of Examples 1 and 2

The products obtained in Examples 1 and 2, given that the reaction conducted until its completion, were subjected to distillation. The lowest-boiling products are HCl, acetic acid, and possible traces of water, and consequently these were removed from the mixture. The residue obtained in this way was subjected to an analysis of verification, and its composition for the two cases considered is provided in Table 3 below.

**Table 3 - Analysis of the product of reaction after purification by distillation of the low-boiling components**

| Sample | alpha | beta | alpha gamma | alpha beta | glycerol |
|---|---|---|---|---|---|
| Table 1 | 0 | 5.8 | 82.1 | 12.1 | 0 |
| Table 2 | 0 | 3.9 | 92.2 | 3.9 | 0 |

### References

[1] Solvay Inc. Process for the production of alpha-epichlorhydrin. GB 799, 567 (1958).
[2] P. Bruin; Epoxy ethers, their polymers and derivatives. GB 814,695(1959).
[3] Z. Brojer, P. A. Penczec,; J. H. Sas; Process of manufacturing epoxide resins. GB 1,001,364, (1965)
[4] T. Kawabe, M. Kadoma, K. Murakami, H. Itoh; Process for continuous production of epichlorohydrin; US 4113746 (1978)
[5] S. Carrà, E. Santacesaria, and M. Morbidelli, Ind. Eng. Chem. Process Des. Dev., 18 (3), 424-427, (1979).
[6] S. Carrà, E. Santacesaria, and M. Morbidelli, Ind. Eng. Chem. Process Des. Dev., 18 (3), 428-433, (1979)
[7] Clarke, H. T.; Hartman, W. W. Epichlorohydrin. In Organic Syntheses, Coll.; (1941); Vol. 1, p 233.
[8] P. Krafft, P. Gilbeau, B. Gosselin, S. Claessens, (Solvay). Process for producing dichloropropanol from glycerol, the glycerol coming eventually from the conversion of animal fats in the manufacture of biodiesel. PCT Patent, WO 054167 A1, 2005.
[9] Siano, D.; Santacesaria, E.; Fiandra, V.; Tesser, R.; Di Nuzzi, G.; Di Serio, M.; Nastasi, M.. (Eurochem Engineering srl); Process for the production of α,γ-dichlorohydrin from glycerol and hydrochloric acid. WO 111810 A2, 2006, US 2009/0062574, EP 1879842 B1
[10] D. Schreck, W. Kruper, R. D. Varjian, M. E. Jones, R. M. Campbell, K. Kearns, B. D. Hook, , J. R. Briggs, J. G. Hippler, (Dow Global Tech. Inc.). Conversion of multihydroxylated-aliphatic hydrocarbon or ester thereof to a chlorohydrin. PCT Patent, WO 2006/020234 and EP 1 771 403 B1 (2007)
[11] S. Cassarino, F. Simola; (CONSER SpA) Conversion of glycerol to dichlorohydrins and epichlorohydrin. WO 2009/066327
[12] J. B. Conant, O. R. Quayle; Glycerol αγ-dichlorohydrin. In Organic Syntheses, Coll.; (1941); Vol. 1, p 292.
[13] J. B. Conant, O. R. Quayle; Glycerol α-monochlorohydrin. In Organic Syntheses, Coll.; (1941); Vol. 1, p 294.
[14] Fauconnier, Sanson, Bull. Soc. Chim., 48, (1887), pp. 236-240
[15] A. J. Hill, E. J. Fischer, A synthesis of beta-chloro-allyl chloride; J. Am. Chem. Soc., (1922) Vol. 44, pp. 2582-2595
[16] E. G. Britton, R. X. Heindel, Preparation of glycerol dichlorohydrin; US 2,144,612 (1939)
[17] E. C. Britton, H. R. Slagh; Glycerol dichlorohydrin; US 2,198,600 (1940)
[18] W. P. Thompson; Recovery of aliphatic chlorohydrins having from 3 to 5 carbon atoms from aqueous solution. GB 931,211 (1963).
[19] R. Tesser, M. Di Serio, R. Vitiello, V. Russo, E. Ranieri, E. Speranza, E. Santacesaria; Glycerol Chlorination in Gas_Liquid Semibatch Reactor: An Alternative Route for Chlorohydrins Production; Ind. Eng. Chem. Res. 51 (2012) 8768-8776.
[20] B. M. Bell, J. R. Briggs, R. M. Campbell, S. M. Chambers, P. D. Gaarenstroom, J. G. Hippler, B. D. Hook, K. Kearns, J. M. Kenney, W. J. Kruper, D. J. Schreck, C. N. Theriault, C. P. Wolfe, Glycerol as a Renewable Feedstock for Epichlorohydrin Production. The GTE Process, Clean 36 (2008) 657 - 661
[21] E. Santacesaria, R. Tesser, M. Di Serio, L. Casale, D. Verde, New Process for Producing Epichlorohydrin via Glycerol Chlorination; Ind. Eng. Chem. Res. 49 (2010) 964-970.
[22] R. Tesser, E. Santacesaria, M. Di Serio, G. Di Nuzzi, and V. Fiandra; Kinetics of Glycerol Chlorination with Hydrochloric Acid: A New Route to αγ-Dichlorohydrin; Ind. Eng. Chem. Res. 46 (2007) 6456-6465.
[23] E. Santacesaria, M. Di Serio, R. Tesser, (Eurochem Engineering srl). Process for monochlorohydrins production from glycerol and hydrochloric acid. PCT Patent WO 132770 A1, 2008.
[24] E. Santacesaria, R. Tesser, R. Vitiello, M. Di Serio; (Eurochem Engineering srl) Processo per la produzione di cloridrine; Italian Patent RM2013A000488

## Claims

1. A process for the production of dichlorohydrins comprising the stage of getting glycerol to react directly with a mono-, bi-, or polyfunctional acyl chloride in a stoichiometric amount.

2. The process according to Claim 1, wherein acyl chloride has the following formula (I) 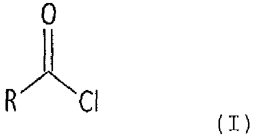 where R is a linear, branched, or cyclic alkyl chain with 2 to 10 carbon atoms, possibly substituted with one or more groups R', which are the same as or different from one another, chosen from among: a linear, branched, or cyclic aliphatic group C₁-C₁₀; an aromatic group C₁-C₁₀; and one, or more than one, further acyl-chloride group.

3. The process according to any one of Claims 1 and 2, wherein the acyl chloride is chosen from among: acetyl chloride, propionyl chloride, adipoyl dichloride, malonyl dichloride, succinyl dichloride, phenylacetyl chloride.

4. The process according to any one of Claims 1-3, wherein glycerol, whether refined or raw, comes from biodiesel production plants.

5. The process according to any one of Claims 1-4, conducted in the presence of inorganic salts, such as alkaline chlorides, preferably sodium or potassium chlorides, preferably present in amounts of between 1 and 20%, preferably between 5 and 10%.

6. The process according to any one of Claims 1-5, wherein acyl chloride and/or glycerol are supplied continuously.

7. The process according to any one of Claims 1-6, wherein the reaction is carried out at a temperature of between 80 and 250°C, preferably between 100 and 175°C.

8. The process according to any one of Claims 1-7, wherein the reaction is carried out at a pressure of between 0.5 and 50 bar, preferably between 5 and 10 bar.

## Patentansprüche

1. Verfahren zur Herstellung eines Dichlorhydrins, das die Stufe umfasst, Glycerol direkt mit einem mono-, bi- oder polyfunktionalen Acylchlorid in einer stöchiometrischen Menge umzusetzen.

2. Verfahren nach Anspruch 1,
wobei das Acylchlorid die folgende Formel (I) aufweist 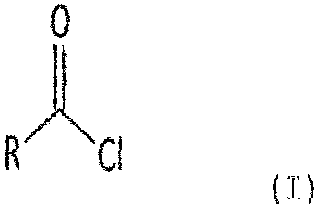 wobei R eine lineare, verzweigte oder zyklische Alkylkette mit zwei bis zehn Kohlenstoffatomen ist, möglicherweise mit einer oder mehreren Gruppen R' substituiert, die gleich oder verschieden sind, ausgewählt aus folgendem:
einer linearen, verzweigten oder zyklischen aliphatischen C₁C₁₀-Gruppe; einer aromatischen C₁-C₁₀-Gruppe; und einer oder mehr als einer weiteren Acyl-Chloridgruppe.

3. Verfahren nach einem der Ansprüche 1 und 2,
wobei das Acylchlorid aus folgendem ausgewählt ist:
Acetylchlorid, Propionylchlorid, Adipoyldichlorid, Malonyldichlorid, Succinyldichlorid, Phenylacetylchlorid.

4. Verfahren nach einem der Ansprüche 1 bis 3,
wobei Glycerol, entweder raffiniert oder roh, aus Biodiesel-Produktions-Fabriken stammt.

5. Verfahren nach einem der Ansprüche 1 bis 4,
durchgeführt in Gegenwart von anorganischen Salzen wie beispielsweise alkalischen Chloriden, bevorzugt Natrium- oder Kaliumchloriden, vorzugsweise vorliegend in Mengen zwischen 1 bis 20 %, vorzugsweise zwischen 5 und 10 %.

6. Verfahren nach einem der Ansprüche 1 bis 5,
wobei Acylchlorid und/oder Glycerol kontinuierlich zugesetzt werden.

7. Verfahren nach einem der Ansprüche 1 bis 6,
wobei die Reaktion bei einer Temperatur von zwischen 80 und 250°C, vorzugsweise zwischen 100 und 175°C durchgeführt wird.

8. Verfahren nach einem der Ansprüche 1 bis 7,
wobei die Reaktion bei einem Druck von zwischen 0,5 und 50 bar, vorzugsweise zwischen 5 und 10 bar durchgeführt wird.

## Revendications

1. Procédé pour la production de dichlorohydrines comprenant l'étape consistant à faire réagir du glycérol directement avec un chlorure d'acyle mono-, bi- ou polyfonctionnel dans une quantité stoechiométrique.

2. Procédé selon la revendication 1, dans lequel le chlorure d'acyle a la formule suivante (I) 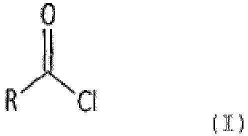 où R est une chaîne alkyle linéaire, ramifiée ou cyclique avec 2 à 10 atomes de carbone, éventuellement substitués par un ou plusieurs groupes R', qui sont les mêmes ou différents l'un de l'autre, choisis parmi : un groupe aliphatique linéaire, ramifié ou cyclique C₁-C₁₀ ; un groupe aromatique C₁-C₁₀ ; et un, ou plus d'un, autre groupe de chlorure d'acyle.

3. Procédé selon l'une quelconque des revendications 1 et 2, dans lequel le chlorure d'acyle est choisi parmi : chlorure d'acétyle, chlorure de prorionyle, dichlorure d'adipoyle, dichlorure de malonyle, dichlorure de succinyle, chlorure de phénylacétyle.

4. Procédé selon l'une quelconque des revendications 1 à 3, dans lequel le glycérol, soit raffiné soit brut, provient d'installations de production de biodiesel.

5. Procédé selon l'une quelconque des revendications 1 à 4, exécuté en présence de sels inorganiques, tels que des chlorures alcalins, de préférence des chlorures de sodium ou potassium, de préférence présents dans des quantités comprises entre 1 et 20%, de préférence entre 5 et 10%.

6. Procédé selon l'une quelconque des revendications 1 à 5, dans lequel le chlorure d'acyle et/ou le glycérol sont alimentés en continu.

7. Procédé selon l'une quelconque des revendications 1 à 6, dans lequel la réaction est exécutée à une température comprise entre 80 et 250°C, de préférence entre 100 et 175°C.

8. Procédé selon l'une quelconque des revendications 1 à 7, dans lequel la réaction est exécutée à une pression comprise entre 0,5 et 50 bar, de préférence entre 5 et 10 bar.
